# Europäisches Patentamt

# European Patent Office

# Office européen des brevets

(11) Veröffentlichungsnummer: **0 173 949**
**B1**

(12) # EUROPÄISCHE PATENTSCHRIFT

(45) Veröffentlichungstag der Patentschrift:
08.11.89

(51) Int. Cl.⁴: **C 07 H 5/02** // C07H13/04

(21) Anmeldenummer: 85110754.0

(22) Anmeldetag: 27.08.85

(54) Verfahren zur einstufigen Herstellung von Glycosylfluoriden.

(30) Priorität: 05.09.84 DE 3432565

(43) Veröffentlichungstag der Anmeldung:
12.03.86 Patentblatt 86/11

(45) Bekanntmachung des Hinweises auf die Patenterteilung:
08.11.89 Patentblatt 89/45

(84) Benannte Vertragsstaaten:
AT BE CH DE FR GB IT LI NL SE

(73) Patentinhaber: HOECHST AKTIENGESELLSCHAFT,
Postfach 80 03 20, D-6230 Frankfurt am Main 80 (DE)

(72) Erfinder: **Franz, Raimund, Dr.,**
**Johann-Strauss-Strasse 36, D-6233 Kelkheim (Taunus)**
**(DE)**
Erfinder: **Deger, Hans-Matthias, Dr., Am Rheingauer**
**Weg 8, D-6238 Hofheim am Taunus (DE)**
Erfinder: **Schlingmann, Merten, Dr., Schneidhainer**
**Strasse 32a, D-6240 Königstein/Taunus (DE)**

(56) Entgegenhaltungen:
CHEMICAL ABSTRACTS, Band 101, Nr. 19, 5. November 1984, Seite 734, Zusammenfassung Nr. 171601e, Columbus, Ohio, US; T. GOTO et al.: "Effect of glycosyl halides on glucose isomerase. I. Synthesis of glycosyl fluorides and their effects on the activity of glucose isomerase", & SHOKUHIN SOGO KENKYUSHO DENKYU HOKOKU 1983, (41), 50-3
CARBOHYDRATE RESEARCH, Band 110, 1982, Seiten 217-227, Elsevier Scientific Publishing Company, Amsterdam, NL; J. DEFAYE et al.: "The behavior of cellulose, amylose, and beta-D-xylan towards anhydrous hydrogen fluoride"

(56) Entgegenhaltungen: (Fortsetzung)
CHEMICAL ABSTRACTS, Band 102, Nr. 2, 14. Januar 1985, Seite 97, Zusammenfassung Nr. 8411e, Columbus, Ohio, US; J. DEFAYE et al.: "Hydrogen fluoride saccharification of cellulose and lignocellulose materials", & J. APPL. POLYM. SCI.: APPL. POLYM. SYMP. 1983, 37(PROC. CELLUL. CONF., 9th, 1982, Part 2), 653-70

Anmerkung· Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art 99(1) Europäisches Patentübereinkommen)

## Beschreibung

Unter den 1-Halogenozuckern (Glycosylhalogeniden) nehmen die Fluorverbindungen auf Grund ihrer relativen Stabilität und besonderen Reaktivität eine Sonderstellung ein. Dies gilt insbesondere für Glycosid-Synthesen, die mit Glycosylhalogeniden durchgeführt werden. Glycosylfluoride sind die einzigen 1-Halogenozucker, die ohne Schutz der übrigen Hydroxygruppen beständig sind, wobei sich allerdings die anomeren Formen in ihrer Stabilität unterscheiden können. So ist z. B. α-Glucosylfluorid stabiler als die β-Form.

Obwohl, wie erwähnt, die Glycosylfluoride ohne Schutzgruppen beständig sind, ist ihre Herstellungsmethode gemäß der Literatur relativ umständlich und verläuft, vom reinen Kohlenhydrat (Monosaccharid) ausgehend, in drei Stufen. Die vorliegende Erfindung betrifft nun eine Methode zur einstufigen Herstellung von 1-Fluorozuckern (Glycosylfluoriden) aus einem Kohlenhydrat.

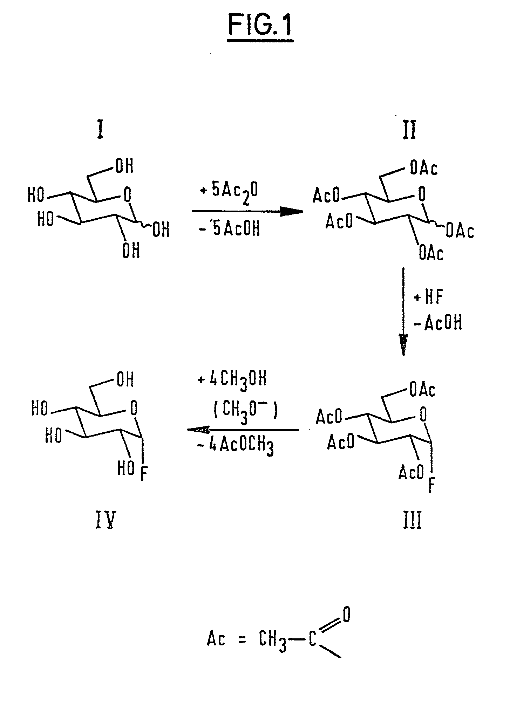

Der Stand der Technik soll am Beispiel der Herstellung von α-Glucosylfluorid dargestellt werden. Hier bedient man sich immer noch der Methode von B. Helferich et al. (Liebigs Annalen der Chemie 447 (1926), S. 27 ff), derzufolge Glucose (I) in Stufe 1 in ihr Pentaacetat (II) übergeführt werden muß. Erst dieses kann in Stufe 2 mittels flüssigem Fluorwasserstoff zu Tetraacetyl-α-glucosyl-fluorid (III) umgesetzt werden, das schließlich in Stufe 3 im Sinne einer Umesterung mit Methanol in Gegenwart von Methylat in das schutzgruppen-freie α-Glucosylfluorid (IV) umgewandelt werden muß (s. Formelschema in Figur 1).

Als zusätzlicher Nachteil fällt ins Gewicht, daß der bei der Fluorierung (Stufe 2) verwendete Fluorwasserstoff verloren geht, hierbei auch teilweise eine Deacetylierung eintreten kann, die gegebenenfalls durch Nachacetylieren behoben werden muß, und daß mit der Abspaltung der Schutzgruppen durch Methylat auch eine Dehydrofluorierung unter Bildung eines Zuckeranhydrids eintreten kann, was die Reinheit des Endproduktes beeinträchtigt und für nachfolgende Reaktionen ungünstig sein kann.

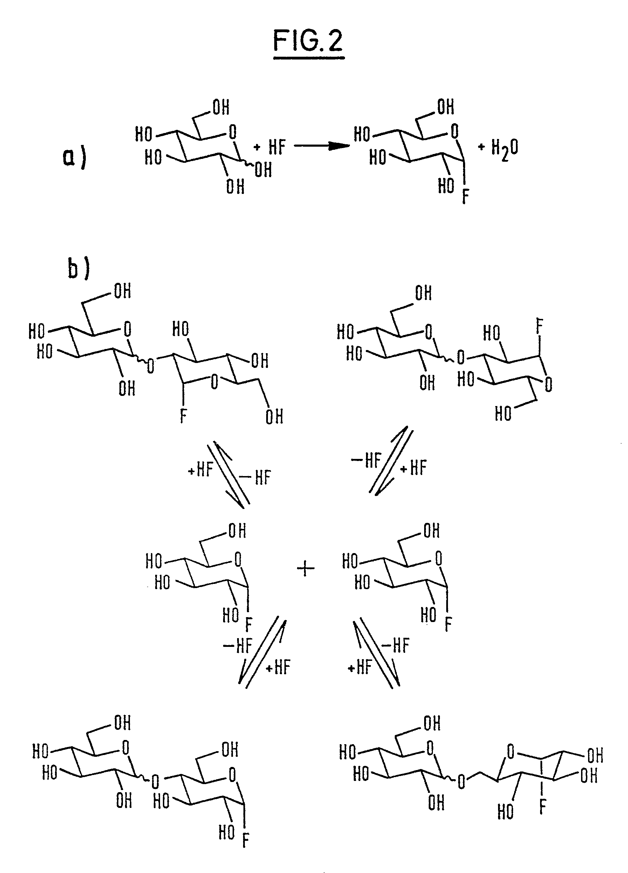

Die relative Stabilität der Glycosylfluoride, z. B. des α-Glucosylfluorids, legt den Gedanken nahe, dieses Produkt in einer Stufe direkt aus dem Monosaccharid, z. B. der Glucose, und Fluorwasserstoff herzustellen. In der Tat ist bekannt, daß Monosaccharide, aber auch Oligo- und Polysaccharide, wie Stärke oder Cellulose, leicht mit Fluorwasserstoff unter Bildung von Glycosylfluoriden reagieren (vgl. Defaye, Gadelle und Pedersen, Carbohydrate Research 110 (1982), S. 217–227, mit weiteren Literaturangaben, siehe Formelschema in Figur 2a). Wie dieser Literaturstelle weiter zu entnehmen ist, steht gebildetes Glucosylfluorid jedoch in einem konzentrationsabhängigen Gleichgewicht mit Reversionsprodukten, d. h. Produkten der Reaktion des Glucosylfluorids mit einer beliebigen Hydroxygruppe eines Nachbarmoleküls gemäß dem Formelschema in Figur 2b. Die dabei entstehenden fluorierten Disaccharide können ebenso im Gleichgewicht mit weiteren mono-, oligo- oder polymeren Saccharidmolekülen im Reaktionsgemisch kondensieren. Wie dieser Literaturstelle auch zu entnehmen ist, wird das Gleichgewicht beim Abdampfen des Fluorwasserstoffs in Richtung auf die Oligosaccharide (Reversionsprodukte) verschoben.

Um die unerwünschte, konzentrationsabhängige Verschiebung der Gleichgewichte in Richtung der Reversionsprodukte zu vermeiden, hat man auch schon Glucosylfluorid aus verdünnter Lösung in Fluorwasserstoff durch Ausfällen mit Diäthyläther isoliert (Defaye, Gadelle, Pedersen, loc. cit.). Diese Methode eignet sich jedoch aus Sicherheitsgründen nicht für größere Mengen (Brand- und Explosionsgefahr im Zusammenhang mit Diäthyläther); außerdem müssen Fluorwasserstoff und Diäthyläther, da sie nur schwer voneinander getrennt werden können, verloren gegeben werden, was diese Methode sehr kostspielig und nur für wissenschaftliche Zwecke brauchbar macht. Eine weitere bekannte, aber für große Mengen ähnlich unpraktikable Methode ist das unmittelbare Neutralisieren der Lösung von Glucosylfluorid mit Calciumcarbonat (D. T. A. Lamport et al., Biotechnology and Bioengineering, Vol. XXIV, Seiten 903–918 (1982)). Dieses Verfahren liefert außerdem nur Ausbeuten von höchstens etwa 20%.

Es bestand daher die Aufgabe, ein Verfahren zur einstufigen Herstellung von Glycosylfluoriden zu entwickeln, das eine Wiedergewinnung des Fluorwasserstoffs erlaubt, kein Fällungsmittel benötigt und dennoch ohne Reversion des primär entstandenen Glycosylfluorids abläuft.

Es wurde nun überraschend gefunden, daß die Geschwindigkeit der Einstellung des erwähnten konzentrationsabhängigen Reversionsgleichgewichtes in Fluorwasserstoff sehr stark temperaturabhängig ist, so daß man eine Reversion von Sacchariden, deren Hydroxylgruppen ungeschützt sind, in einfacher Weise dadurch vermeiden kann, daß man beim Aufarbeiten einer Lösung eines Glycosylfluorids solcher Saccharide in Fluorwasserstoff eine geeignet niedrige Verdampfungstemperatur und/oder eine geeignet hohe Verdampfungsgeschwindigkeit auswählt.

Gegenstand der vorliegenden Erfindung ist somit ein Verfahren zur einstufigen Herstellung von Glycosylfluoriden durch Auflösen von Sacchariden, d. h. Mono-, Oligo- oder Polysacchariden, in flüssigem Fluorwasserstoff und Verdampfen des Fluorwasserstoffs, das dadurch gekennzeichnet ist, daß man von einem Saccharid ausgeht, dessen Hydroxylgruppen ungeschützt sind, und das gebildete Glycosylfluorid in der Weise isoliert, daß bei der Verdampfung bei einer relativ hohen Temperatur bei Beginn der Verdampfung eine hohe Verdampfungsgeschwindigkeit bewirkt bzw. daß beim Arbeiten bei einer niedrigen Verdampfungsgeschwindigkeit bei Beginn der Verdampfung eine ausreichend niedrige Temperatur eingestellt wird.

Mit anderen Worten handelt es sich bei der Erfindung auch um ein Verfahren zur Abtrennung des überschüssigen Fluorwasserstoffs aus dem Reaktionsgemisch von Sacchariden und flüssigem Fluorwasserstoff unter Isolierung der Glycosylfluoride, das dadurch gekennzeichnet ist, daß man von einem Saccharid ausgeht, dessen Hydroxylgruppen ungeschützt sind, und daß der überschüssige Fluorwasserstoff in der Weise verdampft wird, daß bei einer relativ hohen Temperatur bei Beginn der Verdampfung eine hohe Verdampfungsgeschwindigkeit bewirkt bzw, daß beim Arbeiten bei einer niedrigen Verdampfungsgeschwindigkeit bei Beginn der Verdampfung eine ausreichend niedrige Temperatur eingestellt wird.

Bei dem erfindungsgemäßen Verfahren wird das Glycosylfluorid naturgemäß in der stereomeren Form erhalten, die thermodynamisch stabiler ist, gewöhnlich in der α-Form.

Für die Temperatur bei Beginn der Verdampfung kommt der gesamte flüssige Bereich der Lösung des Ausgangsstoffes in Fluorwasserstoff zwischen −80 °C und der Siedetemperatur des Fluorwasserstoffs bei Atmosphärendruck, bei Normaldruck +20 °C, in Betracht. Ein bevorzugter Bereich ist jedoch der zwischen −50 °C und 0 °C, insbesondere der zwischen −40 °C und −20 °C, wobei bei höheren Verdampfungsgeschwindigkeiten höhere Temperaturen bis zu den oberen Grenzen der angegebenen Bereiche zulässig sind und bei kleineren Verdampfungsgeschwindigkeiten zweckmäßig niedrigere Temperaturen eingehalten werden, d. h. daß man diese dann in der Nähe der unteren Grenzen der angegebenen Bereiche einstellt.

Unter dem Begriff «relativ hohe Temperatur bei Beginn der Verdampfung» werden Temperaturen im Bereich von −20 bis +20, vorzugsweise von −20 bis 0 °C verstanden und unter dem Begriff «niedrige Temperatur bei Beginn der Verdampfung» solche im Bereich von −80 bis −30, vorzugsweise von −50 bis −30 °C. Da es Ziel der Erfindung ist, die Reversion des Glycosylfluorids zu vermeiden, ist es natürlich auch im Bereich von −50 bis 0 °C bzw. in dem engeren bevorzugten Bereich zwischen −40 und −20 °C bevorzugt, solche Maßnahmen zu ergreifen, die der Reversion entgegenwirken, d. h. eine möglichst hohe Verdampfungsgeschwindigkeit zu bewirken.

Eine niedrige Temperatur bei Beginn der Verdampfung kann z. B. durch eine Destillation unter vermindertem Druck, oder durch eine Verdampfung des Fluorwasserstoffs durch Einblasen von Inertgas (z. B. Luft, Stickstoff, Kohlendioxid oder Edelgase) ohne oder vorzugsweise mit Außenkühlung eingestellt werden. Jedoch kann man auch durch Erzeugen einer so hohen Verdampfungsgeschwindigkeit und damit eines hohen Verbrauchs an Verdampfungswärme pro Zeiteinheit eine so starke innere Kühlung der Lösung bewirken, daß eine Außenkühlung unnötig ist.

Die insbesondere im letzteren Fall nötige hohe Verdampfungsgeschwindigkeit kann z. B. durch die Anwendung üblicher Verfahren, wie der mechanischen Zerstäubung oder Sprühtrocknung, Dünnschicht- oder Fallfilmverdampfung erzeugt werden. Der Eindampfrückstand wird in diesen Fällen zweckmäßig sofort, vorzugsweise kontinu-

ierlich, neutralisiert oder aber vor weiterer Verarbeitung tiefgekühlt, z. B. mittels festem Kohlendioxid.

Fluorwasserstoff und Saccharide sind an sich in jedem Verhältnis mischbar. In den Lösungen, die der erfindungsgemäßen Behandlung unterworfen werden, kann das Verhältnis des Fluorwasserstoffs zum Saccharid z. B. mindestens das 4-fache betragen. Im allgemeinen beträgt es aber mindestens das 7-fache und insbesondere mindestens das 9-fache. Theoretisch kann das Verhältnis sehr groß sein, z. B. das 100-fache betragen, jedoch ist dieses aus Gründen der technischen Handhabung naturgemäß nicht praktisch, so daß man im allgemeinen den Überschuß an Fluorwasserstoff in technisch sinnvoller Weise beschränkt, z. B. auf höchstens das 20- oder 30-fache.

Als Ausgangsstoffe für die erfindungsgemäße Herstellung von Glycosylfluoriden können die monomeren Aldo- und Ketotetrosen, -pentosen, -hexosen, -heptosen, -octosen und -nonosen, aber auch glycosidisch verknüpfte Oligo- und Polysaccharide, deren glycosidische Bindungen mittels Fluorwasserstoff spaltbar sind, verwendet werden. Als Beispiele für die in diesem Sinn einsetzbaren Monosaccharide werden angeführt: Glucose, Galactose, Mannose, Allose, Fructose, Sorbose, Fucose, Rhamnose, Xylose, Ribose, Arabinose und Erythrose. Als Beispiele für glycosidisch verknüpfte Oligo- und Polysaccharide werden angeführt: Maltose, Isomaltose, Cellobiose, Lactose, Saccharose, Raffinose, Cellodextrin, Amylose, Amylopectin und Cellulose, wobei selbstverständlich zur Herstellung eines einheitlich zusammengesetzten Glycosylfluorids nur ein einheitlich aufgebauter Ausgangsstoff dienen kann.

Als Werkstoffe für das Reaktionsgefäß bzw. die Verdampfungsapparatur kommen alle Materialien infrage, die unter den Reaktionsbedingungen gegenüber Fluorwasserstoff beständig sind, wie Polyäthylen, Polypropylen, Copolymere aus Hexafluorpropen und Vinylidenfluorid, z. B. ®Viton, Polytetrafluoräthylen, Polytrifluorchloräthylen, Chromnickelstahl, Reinnickel, Monelmetall, Silber, Gold, Platin. Für Apparateteile minderer Beanspruchung kann als metallischer Werkstoff auch unlegierter Stahl eingesetzt werden.

Der gewählte Ausgangsstoff wird in üblicher Weise unter Rühren in Fluorwasserstoff gelöst; hierbei wird zweckmäßig der Fluorwasserstoff vorgelegt und das feste Saccharid eindosiert. Die dabei freiwerdende Reaktions- und Solvatationswärme kann z. B. durch Außenkühlung oder Rückflußkühlung abgeführt oder als Verdampfungswärme verbraucht werden.

Nach erfolgter Auflösung des als Ausgangsstoff eingesetzten Saccharids ist es zweckmäßig, die Reaktion noch einige Zeit, z. B. etwa 15 bis 200 Minuten, bevorzugt 30 bis 100 Minuten, gegebenenfalls unter Rühren, zweckmäßig im Temperaturbereich von 0 bis 20 °C zu vervollständigen. Anschließend wird die klare Reaktionslösung erfindungsgemäß dem Abdampfprozeß unterworfen.

Der beim erfindungsgemäßen Verfahren abgedampfte Fluorwasserstoff kann, nach der Kondensation in einem geeigneten Kühler, in der Regel unmittelbar wiederverwendet werden. Die Ausbeute an zurückgewonnenem Fluorwasserstoff liegt, je nach verwendeter Eindampfmethode, bis zu weit über 90% des Einsatzes.

Der gegebenenfalls gekühlte Eindampfrückstand, der das rohe Glycosylfluorid, Restmengen an Fluorwasserstoff sowie gegebenenfalls Reaktionswasser enthält, kann wiederum in üblicher Weise, z. B. mittels Calciumcarbonat in wäßriger Aufschlämmung (vgl. D. T. A. Lamport et al., loc. cit.), neutralisiert werden. Die Isolierung des 1-Fluorzuckers aus dem neutralen, wäßrigen Filtrat kann z. B. durch Eindampfen erfolgen. Bevorzugt werden jedoch schonende Methoden, wie die Gefriertrocknung. Die Ausbeuten sind, da Verluste nur im Zusammenhang mit einem einzigen Filtrationsvorgang auftreten können, stets sehr hoch, in der Regel nahezu quantitativ. Das so erhaltene Produkt ist meist für die weitere Verwendung ausreichend rein. Da die Glycosylfluoride sich in ihrer Stabilität etwas unterscheiden, tritt zuweilen das entsprechende fluorfreie Monosaccharid, z. B. die Galactose, in geringen Mengen, z. B. bis zu 5%, als Verunreinigung auf. Zur Identifikation und Reinheitskontrolle eignet sich neben der Elementaranalyse vor allem das Dünnschichtchromatogramm.

Das erfindungsgemäße Verfahren hat gegenüber dem Stand der Technik folgende beiden großen Vorteile:

a) Es handelt sich um eine einstufige Reaktion, ausgehend von einem an den Hydroxylgruppen ungeschützten Kohlenhydrat, welches nicht nur ein Monosaccharid, sondern auch ein preiswertes polymeres Saccharid, nämlich ein Oligo- oder Polysaccharid, wie Stärke oder Cellulose, sein kann; dabei werden die polymeren Saccharide zu monomeren bzw. oligomeren Produkten abgebaut.

b) Fast der ganze, relativ kostspielige Fluorwasserstoff kann zurückgewonnen und wiederverwendet werden.

Beide Vorteile bewirken, daß Glycosylfluoride auf die erfindungsgemäße Weise schneller, einfacher und billiger hergestellt werden können als bisher.

Die folgenden Beispiele sollen das Verfahren weiter erläutern.

Beispiel 1

In einem halbtransparenten, verschließbaren Gefäß aus Polyäthylen mit Magnetrühreinrichtung und Thermometer wurden 50 g Fluorwasserstoff vorgelegt und mittels eines Trockeneisbades auf −50 °C abgekühlt; anschließend wurde das Kühlbad entfernt. Unter Überlagerung mit trockenem Stickstoff wurden darauf 5 g Glucose unter Rühren rasch so eingetragen, daß die Endtemperatur etwa +5 °C erreichte. Die so erhaltene, klare, nur schwach gefärbte Reaktionslösung wurde unter gelegentlichem Rühren 1 Stunde bei Raumtemperatur verschlossen stehen gelassen und anschließend auf −30 °C abgekühlt. Mittels eines Gaseinleitungsrohres aus Polyäthylen wurde nun ein Strom trockenen Stickstoffs mit einer Geschwindigkeit von ca. 100 Liter je Stunde in die Lösung geleitet und währenddessen die Temperatur des äußeren Kühlbades so einreguliert, daß eine Innentemperatur von etwa −30 °C erhalten blieb. Der Gasstrom wurde in einen mit Trockeneis beschickten Kühler geleitet, in welchem der Fluorwasserstoff kondensierte und in einer gekühlten Vorlage gesammelt werden konnte. Nach 1,5 Stunden wurde der nunmehr zähe Sirup unter pH-Kontrolle mit einer wäßrigen Aufschlämmung von fein gepulvertem Calciumcarbonat vermengt. Das Reaktionsprodukt wurde schließlich mit wenigen Tropfen einer gesättigten wäßrigen Calciumhydroxidlösung bis zum bleibenden pH 7,5 versetzt und die Flüssigkeit abgesaugt. Das klare Filtrat wurde gefriergetrocknet und lieferte 4,6 g rohes Glucosylfluorid.

Analyse: Der Schmelzbereich betrug 115–118 °C (Zers.) in guter Übereinstimmung mit der Literatur; eine Depression trat beim Mischen mit authentischem Material nicht ein.

Elementaranalyse: Fluor gef. 10,5%; ber. 10,4% Dünnschichtchromatographie auf Kieselgel G, Laufmittel Äthylacetat/Isopropanol/Wasser (23 : 65 : 12): kein Unterschied im Vergleich mit authentischem Material.

Die $^1$H-, $^{19}$F- und $^{13}$C-Kernresonanzspektroskopie beweist das Vorliegen des α-Anomeren.

Beispiel 2

In einem Destillierkolben aus Polytetrafluoräthylen wurde eine analog Beispiel 1 hergestellte Lösung von 10 g Glucose in 100 g Fluorwasserstoff bei einer Innentemperatur von −30 °C unter vermindertem Druck destilliert. Der Druck wurde zwischen 100 und 130 mbar so einreguliert, daß eine stetige Destillation erfolgte. Nach 2,5 Stunden betrug der Rest-HF-Gehalt im Destillationssumpf nur noch 5 g. Die Destillation wurde abgebrochen und der Rückstand wie nach Beispiel 1 aufgearbeitet. Es wurden 9,5 g Glucosylfluorid erhalten.

Beispiel 3

In einer Apparatur und nach dem Verfahren gemäß Beispiel 1 wurde 1 g D-Galactose mit 10 g Fluorwasserstoff umgesetzt, das Gemisch eine Stunde bei −30 bis −40 °C stehen gelassen und der Fluorwasserstoff danach durch Einblasen von Stickstoff in 15 Minuten verjagt. Die Aufarbeitung lieferte α-D-Galactosylfluorid (ca. 1 g), das durch Elementaranalyse, Dünnschichtchromatogramm und $^{19}$F bzw. $^{13}$C-Kernresonanzspektren identifiziert wurde (vgl. J. E. C. Barnett et al., Biochem J. 105 (1967), S. 669). Schmelzpunkt: 130° (Zers.).

Beispiel 4

Ebenso, wie in Beispiel 3 beschrieben, wurde 1 g D-Mannose mit 10 g Fluorwasserstoff umgesetzt. Es wurde ca. 1 g α-D-Mannosylfluorid isoliert und durch Vergleich mit authentischem Material (vgl. Barnett et al., loc. cit. in Beispiel 3) identifiziert.

**Beispiel 5**

In einer Apparatur und nach dem Verfahren gemäß Beispiel 1 wurden 5 g Stärke mit 50 g Fluorwasserstoff umgesetzt. Das Abblasen des Fluorwasserstoffs erfolgt nach einer einstündigen Verweilzeit bei − 30 bis − 35 °C innerhalb einer Stunde. Danach betrug der Rest-Fluorwasserstoffgehalt im Abdampf-Rückstand noch 5 g. Die Neutralisation mit Calciumcarbonat, wie beschrieben, und Filtration unter Verwendung von Cellulosepulver als Filterhilfsmittel führte zu gefriergetrocknetem α-Glucosylfluorid. – Aubeute 4,2 g.

**Beispiel 6**

In einer Apparatur und nach dem Verfahren gemäß Beispiel 1 wurden 5 g Cellulose mit 50 g Fluorwasserstoff umgesetzt. Das Abblasen des Fluorwasserstoffs erfolgte bei − 30 °C innerhalb · von 1,5 Stunden. Danach betrug der Rest-HF-Gehalt im Abdampfrückstand noch 5 g. Die Neutralisation mit Calciumcarbonat, wie beschrieben, Filtration und Gefriertrocknung ergab α-Glucosylfluorid in einer Ausbeute von 4,5 g.

**Beispiel 7**

In einer Apparatur und nach dem Verfahren gemäß Beispiel 1 wurden 5 g D-Glucose mit 50 g Fluorwasserstoff umgesetzt. Nach Ablauf der Nachrührzeit wurde die klare Lösung in einen Tropftrichter aus Polytrifluorchloräthylen übergeführt und von dort in eine zylindrische Kammer aus halbtransparentem Polyäthylen eingetropft, in welcher ein rasch rotierender Blattrührer für eine feine Zerstäubung der Lösung sorgte. Der entweichende Fluorwasserstoff wurde mittels eines aufwärts gerichteten Stickstoff-Stromes (300 l/ Stunde) aus der Kammer geführt und in einem Kühler kondensiert. An der Wand der Kammer sorgte eine schwache Außenheizung durch Warmwasser von 40 °C für eine rasche Verdampfung des Fluorwasserstoffs aus dem ablaufenden Sirup-Film. Der sirupöse Eindampfrückstand lief aus dem gekühlten Boden der Kammer kontinuierlich in eine mit Trockeneis gekühlte Vorlage. Die Dauer der Verdampfung betrug 15 Minuten. Der Eindampfrückstand enthielt noch 10 g Fluorwasserstoff und wurde, wie in Beispiel 1 beschrieben, aufgearbeitet. Die Ausbeute an α-Glucosylfluorid betrug 4,6 g.

**Patentansprüche**

1. Verfahren zur einstufigen Herstellung von Glycosylfluoriden durch Auflösen von Sacchariden in flüssigem Fluorwasserstoff und Verdampfen des Fluorwasserstoffs, dadurch gekennzeichnet, daß man von einem Saccharid ausgeht, dessen Hydroxylgruppen ungeschützt sind, und das gebildete Glycosylfluorid in der Weise isoliert, daß bei der Verdampfung des Fluorwasserstoffs bei einer relativ hohen Temperatur bei Beginn der Verdampfung eine hohe Verdampfungsgeschwindigkeit bewirkt bzw. daß beim Arbeiten bei einer niedrigen Verdampfungsgeschwindigkeit bei Beginn der Verdampfung eine ausreichend niedrige Temperatur eingestellt wird.

2. Verfahren zur Abtrennung des überschüssigen Fluorwasserstoffs aus dem Reaktionsgemisch von Sacchariden und flüssigem Fluorwasserstoff unter Isolierung der Glycosylfluoride, dadurch gekennzeichnet, daß man von einem Saccharid ausgeht, dessen Hydroxylgruppen ungeschützt sind, und daß der überschüssige Fluorwasserstoff in der Weise verdampft wird, daß bei einer relativ hohen Temperatur bei Beginn der Verdampfung eine hohe Verdampfungsgeschwindigkeit bewirkt bzw. daß beim Arbeiten bei einer niedrigen Verdampfungsgeschwindigkeit bei Beginn der Verdampfung eine ausreichend niedrige Temperatur eingestellt wird.

3. Verfahren nach Anspruch 1 oder 2, dadurch gekennzeichnet, daß die Temperatur bei Beginn der Verdampfung im Bereich von − 80 bis + 20, vorzugsweise von − 50 bis 0 und insbesondere von − 40 bis − 20 °C liegt.

4. Verfahren nach einem oder mehreren der Ansprüche 1 bis 3, dadurch gekennzeichnet, daß die relativ hohe Temperatur bei Beginn der Verdampfung im Bereich von − 20 bis + 20, vorzugsweise von − 20 bis 0 °C liegt.

5. Verfahren nach einem oder mehreren der Ansprüche 1 bis 3, dadurch gekennzeichnet, daß die niedrige Temperatur bei Beginn der Verdampfung im Bereich von − 80 bis − 30, vorzugsweise von − 50 bis − 30 °C liegt.

6. Verfahren nach einem oder mehreren der Ansprüche 1 bis 5, dadurch gekennzeichnet, daß bei Beginn der Verdampfung eine niedrige Temperatur durch Destillation unter vermindertem Druck oder durch Einblasen von Inertgas eingestellt wird.

7. Verfahren nach einem oder mehreren der Ansprüche 1 bis 5, dadurch gekennzeichnet, daß eine hohe Verdampfungsgeschwindigkeit durch eine mechanische Zerstäubung, Sprühtrocknung, Dünnschicht- oder Fallfilmverdampfung erzeugt wird.

8. Verfahren nach einem oder mehreren der Ansprüche 1 bis 7, dadurch gekennzeichnet, daß der Verdampfung eine Lösung des Glycosylfluorids unterworfen wird, in der das Gewichtsverhältnis von Fluorwasserstoff zum Saccharid mindestens 4 : 1, vorzugsweise mindestens 7 : 1 und insbesondere mindestens 9 : 1 beträgt.

9. Verfahren nach einem oder mehreren der Ansprüche 1 bis 8, dadurch gekennzeichnet, daß das Glycosylfluorid ein unter Abbau erhaltenes monomeres Umsetzungsprodukt eines polymeren Saccharids, insbesondere eines Polysaccharids ist.

10. Verfahren nach einem oder mehreren der Ansprüche 1 bis 9, dadurch gekennzeichnet, daß die Umsetzung in einer Vorrichtung ausgeführt wird, deren Teile, die mit Fluorwasserstoff in Berührung kommen, aus einem gegen Fluorwasserstoff beständigen Material bestehen.

**Claims**

1. A process for the single-step preparation of glycosyl fluorides by dissolving saccharides in liquid hydrogen fluoride and evaporating the hydrogen fluoride, which comprises starting from a saccharide, the hydroxyl groups of which are unprotected, and isolating the resulting glycosyl fluoride in such a way that a high rate of evaporation is effected at the start of evaporation in the case of evaporation of the hydrogen fluoride at a relatively high temperature or that a sufficiently low temperature is set at the start of evaporation when working with a low rate of evaporation.

2. A process for separating the excess hydrogen fluoride from the reaction mixture of saccharides and liquid hydrogen fluoride with isolation of the glycosyl fluorides, which comprises starting from a saccharide, the hydroxyl groups of which are unprotected, and evaporating the excess hydrogen fluoride in such a way that a high rate of evaporation is effected at the start of evaporation in the case of a relatively high temperature or that a sufficiently low temperatur is set at the start of evaporation when working with a low rate of evaporation.

3. The process as claimed in claim 1 or 2, wherein the temperature at the start of evaporation is in the range from −80 to +20, preferably from −50 to 0 and especially from −40 to −20 °C.

4. The process as claimed in one or more of claims 1 to 3, wherein the relatively high temperature at the start of evaporation is in the range from −20 to +20, preferably from −20 to 0 °C.

5. The process as claimed in one or more of claims 1 to 3, wherein the low temperature at the start of evaporation is in the range from −80 to −30, preferably from −50 to −30 °C.

6. The process as claimed in one or more of claims 1 to 5, wherein a low temperature at the start of evaporation is set by distillation under reduced pressure or by blowing in an inert gas.

7. The process as claimed in one or more of claims 1 to 5, wherein a high rate of evaporation is generated by mechanical atomization, spray-drying, thin-layer evaporation or falling-film evaporation.

8. The process as claimed in one or more of claims 1 to 7, wherein a solution of the glycosyl fluoride, in which the hydrogen fluoride/saccharide weight ratio is at least 4 : 1, preferably at least 7 : 1 and especially at least 9 : 1, is subjected to evaporation.

9. The process as claimed in one or more of claims 1 to 8, wherein the glycosyl fluoride is a monomeric reaction product, obtained with degradation, of a polymeric saccharide, in particular a polysaccharide.

10. The process as claimed in one or more of claims 1 to 9, wherein the reaction is carried out in equipment, of which those components which come into contact with hydrogen fluoride are composed of a material which is resistant to hydrogen fluoride.

**Revendications**

1. Procédé pour préparer en une seule étape des fluorures de glycosyles, par dissolution de saccharide dans du fluorure d'hydrogène liquide et évaporation du fluorure d'hydrogène, procédé caractérisé en ce qu'on part d'un saccharide dont les groupes hydroxyles ne sont pas protégés et en ce qu'on soumet le fluorure de glycosyle ainsi formé à un isolement réalisé en appliquant, lors de l'évaporation du fluorure d'hydrogène à une température relativement élevée, une vitesse élevée d'évaporation au début de cette évaporation ou bien, quand on travaille à une basse température d'évaporation, on ajuste au début de l'évaporation la température à une valeur suffisamment basse.

2. Procédé pour séparer l'excès de fluorure d'hydrogène du mélange réactionnel de saccharides et de fluorure d'hydrogène liquide avec isolement des fluorures de glycosyles, procédé caractérisé en ce qu'on part d'un saccharide dont les groupes hydroxyles ne sont pas protégés et en ce qu'on évapore l'excès de fluorure d'hydrogène de façon qu'à une température relativement élevée on provoque au début de l'évaporation une vitesse élevée d'évaporation ou bien, quand on travaille à une faible vitesse d'évaporation, on ajuste au début de l'évaporation la température à une valeur basse suffisante.

3. Procédé selon la revendication 1 ou 2, caractérisé en ce qu'au début de l'évaporation la température se situe entre −80 et +20°, avantageusement entre −50 et 0° et en particulier entre −40 et −20 °C.

4. Procédé selon une ou plusieurs des revendications 1 à 3, caractérisé en ce que la température relativement élevée se situe, au début de l'évaporation, entre −20 et +20°, avantageusement entre −20 et 0 °C.

5. Procédé selon une ou plusieurs des revendications 1 à 3, caractérisé en ce qu'au début de l'évaporation la basse température se situe entre −80 et −30, avantageusement entre −50 et −30 °C.

6. Procédé selon une ou plusieurs des revendications 1 à 5, caractérisé en ce qu'au début de l'évaporation, on obtient une basse température en effectuant la distillation sous pression réduite ou bien en insufflant un gaz inerte.

7. Procédé selon une ou plusieurs des revendications 1 à 5, caractérisé en ce qu'on obtient une vitesse élevée d'évaporation grâce à une pulvérisation mécanique, un séchage par atomisation, une évaporation en couche mince ou une évaporation dans un appareil à chute de pellicule.

8. Procédé selon une ou plusieurs des revendications 1 à 7, caractérisé en ce qu'on soumet à l'évaporation une solution de fluorure de glycosyle dans laquelle le rapport pondéral du fluorure d'hydrogène au saccharide est au moins égal à 4 : 1, avantageusement au moins égal à 7 : 1 et en particulier au moins égal à 9 : 1.

9. Procédé selon une ou plusieurs des revendications 1 à 8, caractérisé en ce que le fluorure de glycosyle est un produit monomère, obtenu par dégradation, de la réaction de transformation d'un saccharide polymère, en particulier d'un polysaccharide.

10. Procédé selon une ou plusieurs des revendications 1 à 9, caractérisé en ce qu'on conduit la réaction dans un appareillage dont les parties venant au contact du fluorure d'hydrogène consistent en un matériau pouvant résister au fluorure d'hydrogène.

# FIG.1

Ac = $CH_3-C \underset{}{\overset{\displaystyle O}{\diagup}}$

# FIG.2